# EUROPÄISCHE PATENTSCHRIFT

(11) **EP 1 240 872 B1**
(45) Veröffentlichungstag und Bekanntmachung des Hinweises auf die Patenterteilung: **07.12.2005**
(21) Anmeldenummer: 02004652.0
(22) Anmeldetag: 28.02.2002
(51) Int. Cl.: A61B 17/14

(54) **Chirurgisches Sägeblatt mit Ausnehmungen im Arbeitsbereich**
Surgical saw with holes in the cutting portion
Scie chirurgicale avec partie coupante perforée

(30) Priorität: 14.03.2001 DE 10112288
(43) Veröffentlichungstag der Anmeldung: 18.09.2002
(73) Patentinhaber: Gebr. Brasseler GmbH & Co. KG, D-32657 Lemgo (DE)
(72) Erfinder: Danger, Karl-Heinz, 32758 Detmold (DE); Küllmer, Michael, 32657 Lemgo (DE)
(74) Vertreter: Weber, Joachim

(56) Entgegenhaltungen:
- US-A- 4 513 742
- US-A- 5 306 285

## Beschreibung

Die Erfindung bezieht sich auf ein chirurgisches Sägeblatt mit einem Einspannbereich und einem mit einer Verzahnung versehenen Arbeitsbereich. Chirurgische Sägeblätter der beschriebenen Art sind aus dem Stand der Technik in unterschiedlichsten Ausgestaltungsformen bekannt. Sie werden an einer Antriebseinheit montiert, durch welche sie in eine oszillierende, hin- und herschwingende Bewegung versetzbar sind. Hierdurch ist es möglich, beispielsweise präzise Knochenschnitte durchzuführen. Die Sägeblätter können entweder manuell geführt werden, es ist jedoch auch möglich, diese in Zusammenhang mit Schablonen zu verwenden, um präzise vorgegebene Schnitte zu erzeugen.

Durch die flächige Ausgestaltung der Sägeblätter verdecken diese bei bestimmten Arbeitsgängen den Arbeitsbereich, so dass es schwierig oder unmöglich ist, den Sägevorgang optisch zu überwachen. Aus diesem Grunde wurde bereits vorgeschlagen, den Arbeitsbereich mit einer großen Ausnehmung zu versehen, so dass die Verzahnung nur über seitliche Randbereiche mit dem Einspannbereich verbunden ist. Derartige Konstruktionen sind jedoch sehr labil und weisen nicht die erforderliche Festigkeit und Stabilität auf. Eine exakte Schnittführung ist nicht möglich.

Derartige Sägeblätter sind aus der US-A-4 513 742 oder der US-A-5 306 285 bekannt.

Der Erfindung liegt die Aufgabe zugrunde, ein chirurgisches Sägeblatt der eingangs genannten Art zu schaffen, welches bei einfachem Aufbau und kostengünstiger Herstellbarkeit die optische Überwachung des Schnittvorgangs ermöglicht und sich durch eine hohe Stabilität und Festigkeit auszeichnet.

Erfindungsgemäß wird die Aufgabe durch die Merkmale des Hauptanspruchs gelöst, die Unteransprüche zeigen weitere vorteilhafte Ausgestaltungen der Erfindung.

Erfindungsgemäß ist somit vorgesehen, dass in dem Arbeitsbereich eine Vielzahl von Ausnehmungen ausgebildet sind.

Das erfindungsgemäße Sägeblatt zeichnet sich durch eine Reihe erheblicher Vorteile aus. Durch die Vielzahl von Ausnehmungen wird die mechanische Festigkeit des Sägeblattes nicht beeinträchtigt. Vielmehr ergeben sich, wie nachfolgend noch erläutert wird, zusätzliche Steigerungen der Festigkeit. Bedingt durch die oszillierende Bewegung des Sägeblattes ist es möglich, durch die Vielzahl von kleinen Ausnehmungen den Arbeitsbereich in hervorragender Weise optisch zu überwachen und somit den Arbeitsvorgang selbst kontrolliert durchzuführen.

Erfindungsgemäβ since die Ausnehmungen in Form einer Wabenstruktur angeordnet sind. Eine derartigen geometrische Form sichert ein hohes Maß an Festigkeit und Stabilität, da Wabenstrukturen aus sich selbst mit einem minimalen Materialaufwand sehr stabil sind.

Die Wabenstruktur ist erfindungsgemäß so ausgebildet, dass sie von Stegen begrenzte Ausnehmungen umfasst. Die Ausnehmungen können bevorzugterweise sechseckig, rechteckig oder rund oder in sonstiger geometrischer Ausgestaltung vorgesehen sein.

Besonders vorteilhaft ist es, wenn sich die Ausnehmungen im Wesentlichen über die gesamte Breite des Arbeitsbereichs erstrecken, da dann das Sägeblatt das geringste Maß an optischer Abdeckung des Arbeitsbereichs aufweist.

Da es insbesondere wichtig sein kann, direkt den Schnittbereich des Sägeblattes während des Sägevorganges zu beobachten, kann es sich als besonders günstig erweisen, wenn die Ausnehmungen sich von der Verzahnung aus erstrecken.

Weiterhin kann es günstig sein, die Ausnehmungen über einen wesentlichen Teil der Länge des Arbeitsbereiches vorzusehen, um den nicht einsehbaren Teilbereich möglichst gering zu halten.

Im Hinblick auf eine maximale mechanische Festigkeit des Sägeblattes kann es weiterhin besonders vorteilhaft sein, die Ausnehmungen symmetrisch zu einer Mittelachse des Sägeblattes anzuordnen.

Im Folgenden wird die Erfindung anhand von Ausführungsbeispielen in Verbindung mit der Zeichnung beschrieben. Dabei zeigt:
- Fig. 1: eine Seitenansicht eines ersten Ausführungsbeispiels des erfindungsgemäßen Sägeblatts,
- Fig. 2: eine stirnseitige Ansicht des in Fig. 1 gezeigten Sägeblatts,
- Fig. 3: eine Seitenansicht eines weiteren Ausführungsbeispiels eines erfindungsgemäßen Sägeblatts, und
- Fig. 4: eine stirnseitige Ansicht des in Fig. 3 gezeigten Sägeblatts.

In den Figuren sind gleiche Teile mit gleichen Bezugsziffern versehen.

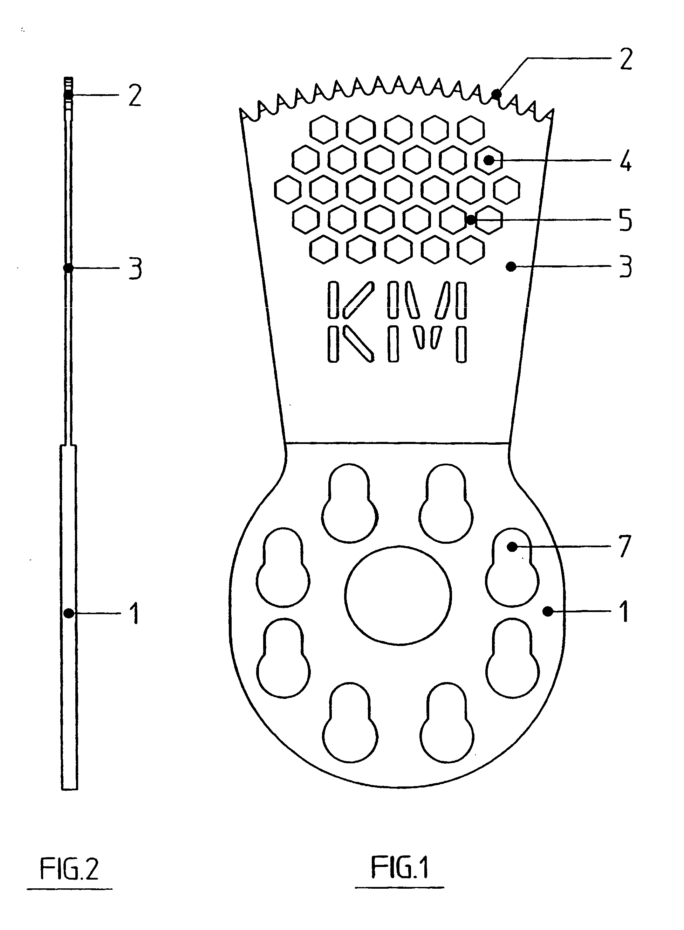

Wie aus Fig. 1 ersichtlich, weist das Sägeblatt einen im Wesentlichen kreisförmigen Einspannbereich 1 auf, welcher mit mehreren Montageöffnungen 7 versehen ist. Sowohl die Montageöffnungen als auch die Dimensionierung des Einspannbereichs 1 sind aus dem Stand der Technik bekannt, so dass auf eine detaillierte Beschreibung verzichtet werden kann. Der Durchmesser des Einspannbereichs 1 beträgt beispielsweise 16 mm.

An den Einspannbereich schließt sich ein Arbeitsbereich 3 an, dessen vorderer, bogenförmiger Rand mit einer Verzahnung 2 versehen ist. Die Verzahnung 2 kann beispielsweise eine Breite von 15 mm haben, die Gesamtlänge des Sägeblattes beträgt beispielsweise 35 mm.

In dem Arbeitsbereich 3 sind eine Vielzahl von sechseckigen, wabenförmig angeordneten Ausnehmungen 4 ausgebildet, die untereinander durch Stege getrennt sind. Die Wabenstruktur weist ein hohes Maß an Festigkeit auf, so dass die Gesamtfestigkeit des Sägeblattes, verglichen mit einem nicht mit Ausnehmungen versehenen Arbeitsbereich praktisch nicht vermindert ist.

Wie sich aus der Seitenansicht der Fig. 2 ergibt, weist der Arbeitsbereich 3 eine geringere Dicke auf, als der Einspannbereich 1 und die Verzahnung 2.

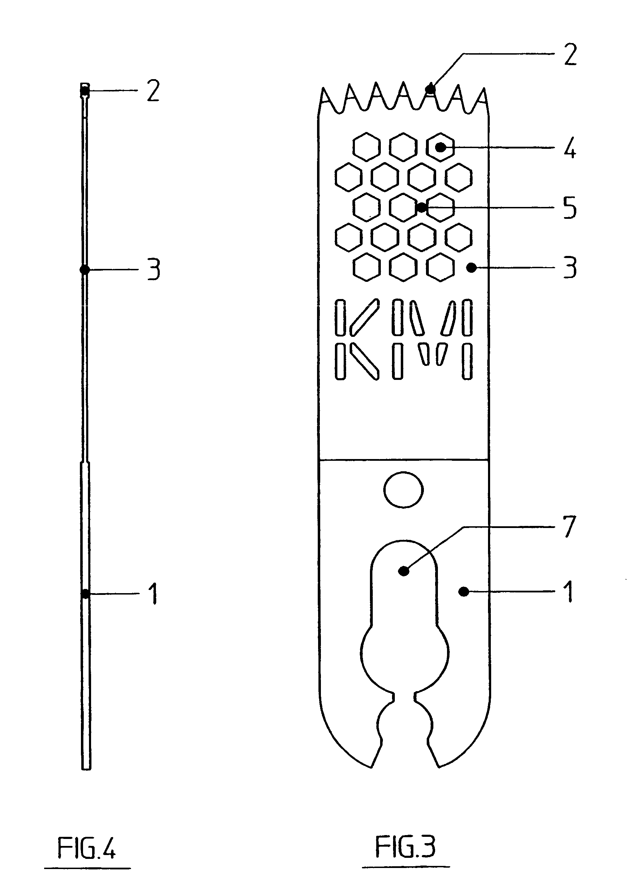

Die Fig. 3 und 4 zeigen ein weiteres Ausführungsbeispiel, bei welchem sowohl der Einspannbereich 1 als auch der Arbeitsbereich 3 durch parallele Außenkanten begrenzt sind, so dass sich eine im Wesentlichen streifenförmige Kontur des Sägeblattes ergibt. Auch bei diesem Ausführungsbeispiel ist die Verzahnung 2 bogenförmig angeordnet, der Mittelpunkt des Kreisbogens der Verzahnung 2 liegt, ebenso wie bei dem Ausführungsbeispiel der Fig. 1, im Drehpunkt bzw. Schwenkpunkt des Sägeblatts.

## Patentansprüche

1. Chirurgisches Sägeblatt, welches die optische Übertragung des Schnittvorgangs ermöglicht, mit einem Einspannbereich (1) und einem mit einer Verzahnung (2) versehenen Arbeitsbereich (3) in welchem Ausnehmungen ausgebildet sind, **dadurch gekennzeichnet, dass** eine Vielzahl von Ausnehmungen (4) in Form einer Wabenstruktur angeordnet sind, welche von Stegen (5) begrenzte Ausnehmungen (4) umfasst.

2. Sägeblatt nach Anspruch 1, **dadurch gekennzeichnet, dass** die Ausnehmungen (4) sechseckig ausgestaltet sind.

3. Sägeblatt nach Anspruch 1 oder 2, **dadurch gekennzeichnet, dass** die Ausnehmungen (4) rechteckig ausgestaltet sind.

4. Sägeblatt nach einem der Ansprüche 1 bis 3, **dadurch gekennzeichnet, dass** die Ausnehmungen (4) rund, dreieckig oder in anderen geometrischen Formen ausgestaltet sind.

5. Sägeblatt nach einem der Ansprüche 1 bis 4, **dadurch gekennzeichnet, dass** die Ausnehmungen (4) im Wesentlichen über die gesamte Breite des Arbeitsbereichs (3) angeordnet sind.

6. Sägeblatt nach einem der Ansprüche 1 bis 5, **dadurch gekennzeichnet, dass** die Ausnehmungen (4) sich von der Verzahnung (2) aus erstrecken.

7. Sägeblatt nach einem der Ansprüche 1 bis 6, **dadurch gekennzeichnet, dass** die Ausnehmungen (4) sich von der Verzahnung (2) aus über einen wesentlichen Teil des Arbeitsbereichs (3) erstrecken.

8. Sägeblatt nach einem der Ansprüche 1 bis 7, **dadurch gekennzeichnet, dass** die Ausnehmungen (4) symmetrisch zu einer Mittelachse (6) des Sägeblatts angeordnet sind.

## Claims

1. Surgical saw blade with a clamping region (1) and with a cutting portion (3) provided with teeth (2) in which cut-outs are formed, **characterised in that** a plurality of cut-outs (4) are arranged in the form of a honeycomb structure comprising cut-outs (4) bordered by webs (5).

2. Saw blade according to claim 1, **characterised in that** the cut-outs (4) have a hexagonal form.

3. Saw blade according to claim 1 or 2, **characterised in that** the cut-outs (4) have a rectangular form.

4. Saw blade according to one of the claims 1 to 3, **characterised in that** the cut-outs (4) have a round, triangular or other geometrical form.

5. Saw blade according to one of the claims 1 to 4, **characterised in that** the cut-outs (4) are arranged over substantially the entire width of the cutting portion (3).

6. Saw blade according to one of the claims 1 to 5, **characterised in that** the cut-outs (4) extend from the teeth (2) outwards.

7. Saw blade according to one of the claims 1 to 6, **characterised in that** the cut-outs (4) extend from the teeth (2) over a substantial proportion of the cutting portion (3).

8. Saw blade according to one of the claims 1 to 7, **characterised in that** the cut-outs (4) are arranged symmetrically about a central axis (6) of the saw blade.

## Revendications

1. Lame de scie chirurgicale permettant le contrôle optique du processus de coupe, avec une zone de serrage (1) et une zone de travail (3) pourvue d'une denture (2) dans laquelle sont réalisés des évidements, **caractérisée en ce qu'**il est disposé un grand nombre d'évidements (4) sous la forme d'une structure en nid d'abeille qui comprend des évidements (4) délimités par des cloisons (5).

2. Lame de scie selon la revendication 1, **caractérisée en ce que** les évidements (4) sont de forme hexagonale.

3. Lame de scie selon la revendication 1 ou 2, **caractérisée en ce que** les évidements (4) sont de forme rectangulaire.

4. Lame de scie selon l'une des revendications 1 à 3, **caractérisée en ce que** les évidements (4) sont circulaires, triangulaires ou présentent d'autres formes géométriques.

5. Lame de scie selon l'une des revendications 1 à 4, **caractérisée en ce que** les évidements (4) sont disposés sensiblement sur toute la largeur de la zone de travail (3).

6. Lame de scie selon l'une des revendications 1 à 5, **caractérisée en ce que** les évidements (4) s'étendent depuis la denture (2).

7. Lame de scie selon l'une des revendications 1 à 6, **caractérisée en ce que** les évidements (4) s'étendent depuis la denture (2) sur une partie essentielle de la zone de travail (3).

8. Lame de scie selon l'une des revendications 1 à 7, **caractérisée en ce que** les évidements (4) sont disposés symétriquement par rapport à un axe médian (6) de la lame de scie.
